# EUROPEAN PATENT SPECIFICATION

(11) **EP 4 084 704 B1**
(45) Date of publication and mention of the grant of the patent: **25.02.2026**
(21) Application number: 20845546.9
(22) Date of filing: 30.12.2020
(51) Int. Cl.: A61B 17/128, A61B 17/42, A61B 17/00, A61B 17/04

(54) **TWO-PIECE CLIP APPLIER JAW ASSEMBLY AND METHOD OF MANUFACTURE**
ZWEITEILIGE CLIP-ANBRINGUNGSBACKENANORDNUNG UND VERFAHREN ZUR HERSTELLUNG
ENSEMBLE MÂCHOIRE D'APPLICATEUR D'AGRAFE EN DEUX PIÈCES ET PROCÉDÉ DE FABRICATION

(30) Priority: 31.12.2019 US 201962955942 P
(43) Date of publication of application: 09.11.2022
(60) Divisional application: 26150781.8
(73) Proprietor: Applied Medical Resources Corporation, Rancho Santa Margarita, CA 92688 (US)
(72) Inventor: SANDSTROM, Alex, La Mirada, CA 90638 (US); HOBBS, Matt, Santa Margarita, CA 92688 (US); GYUGYI, Stephen, Santa Margarita, CA 92688 (US); KINGSBURY, Chase, Santa Margarita, CA 92688 (US); IRWIN, John, Santa Margarita, CA 92688 (US); ELLIOTT, Patrick, Santa Margarita, CA 92688 (US); LEROUX, Mark, Santa Margarita, CA 92688 (US); ARAYA, Matias, Santa Margarita, CA 92688 (US); SHOOK, Mike, Santa Margarita, CA 92688 (US)
(74) Representative: Dolleymores
(86) International application number: PCT/US2020/067573
(87) International publication number: WO 2021/138496

(56) References cited:
- EP-A2- 3 476 315
- WO-A2-2011/028196
- WO-A2-2020/141399
- US-A1- 2011 046 651
- US-A1- 2019 254 679

## Description

### BACKGROUND OF THE INVENTION

### FIELD OF THE INVENTION

The present application relates to surgical instruments and more particularly to a jaw assembly for a surgical clip applier for use in minimally invasive surgical procedures.

### DESCRIPTION OF THE RELATED ART

Endoscopic surgery can frequently require the application of hemostatic clips or the use of other instruments which can ligate, grab, or grip for a variety of purposes. Several significant characteristics of such instruments are simplicity in construction, reliability in operation, as well as low cost. Components that come into contact with internal organs in the body must also be effectively sterilized. Alternatively, the construction can desirably be sufficiently economical to allow disposability of contaminated components. The layout of the instrument should desirably give the surgeon good feedback during the procedure to allow as much control as possible while using the instrument.

Examples of known surgical clip appliers, and methods for manufacturing surgical clip appliers, are disclosed in the following patent documents: US 2019/254679 A1; US 2011/046651 A1; EP 3476315 A2; and WO 2011/028196 A2.

WO2020141399, a document which falls under Article 54(3) EPC, discloses a method to join two clip applier jaws, and corresponding joined clip applier jaws.

Previous hemostatic clip appliers have had various shortcomings. For example, certain clip appliers have had clip jaws with complex geometries requiring multiple manufacturing processes, which added to cost and time of manufacture.

### SUMMARY OF THE INVENTION

According to a first aspect of the present invention there is provided a jaw assembly for a surgical clip applier as recited in claim 1.

According to a second aspect of the present invention there is provided a method of manufacturing a jaw assembly in accordance with the first aspect of the invention.

### BRIEF DESCRIPTION OF THE DRAWINGS

Figure 1 is an exploded view of an embodiment of a laparoscopic clip applier having a jaw assembly in accordance with a first aspect of the present invention;
Figure 2 is an exploded view of a distal end of the laparoscopic clip applier of Figure 1;
Figure 3 is a perspective view of the jaw assembly, in an unassembled state, of the laparoscopic clip applier of Figures 1 and 2;
Figure 4 is a perspective view of the jaw assembly of Figure 3 in an assembled state;
Figure 5 is a schematic view of an exemplary manufacturing process in accordance with the second aspect of the present invention for a jaw of a jaw assembly of a laparoscopic clip applier in accordance with the first aspect of the present invention;
Figure 6 is a schematic view of a first portion of the manufacturing process of Figure 5;
Figure 7 is a schematic view of a second portion of the manufacturing process of Figure 5;
Figure 8 is a schematic view of a third portion of the manufacturing process of Figure 5;
Figure 9 is a schematic view of a fourth portion of the manufacturing process of Figure 5; and
Figure 10 is a schematic view of an exemplary manufacturing process for a jaw assembly.

### DETAILED DESCRIPTION OF THE INVENTION

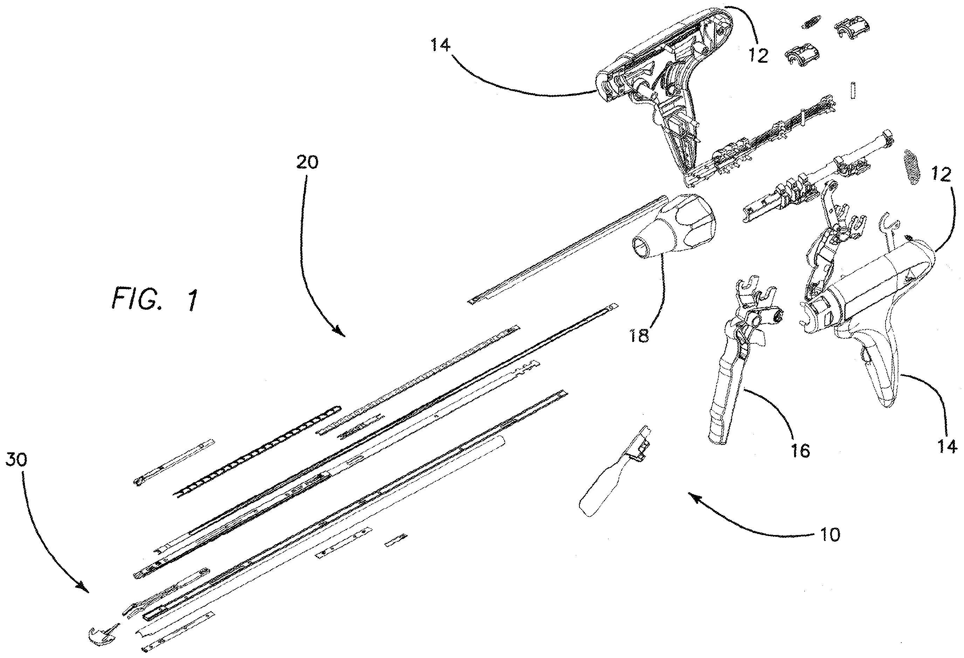

With reference to Figure 1, an exploded view of an embodiment of a laparoscopic clip applier is illustrated. The clip applier comprises a handle assembly 10 at a proximal end thereof. The handle assembly 10 can comprise a handle body 12 extending from a proximal end to a distal end and defining a longitudinal axis of the handle assembly. The handle body 12 has a stationary handle 14 and a movable handle 16 or trigger pivotably coupled to the stationary handle 14. The movable handle 16 can be pivoted from a spaced apart or open configuration relative to the stationary handle 14 to an approximated or closed configuration. The handle assembly 10 further comprises a laparoscopic shaft interface at the distal end of the handle body 12. The laparoscopic shaft interface can have a rotatable collar 18 that is rotatable about the longitudinal axis to rotate a laparoscopic shaft and jaw assembly coupled to the laparoscopic shaft interface. One example of a clip applier device which can incorporate the jaw assemblies further discussed below is described in U.S. Patent No. 10,405,862.

In the illustrated embodiment, the handle assembly 10 comprises a pistol-grip style handle with a stationary handle 14 and a movable handle 16 coupled thereto. But, it is contemplated that in other embodiments, other handle styles can include some or all of the features further described with respect to the illustrated pistol-grip handle. For example, the handle assembly can comprise a scissor-type handle, a generally in-line handle, or another handle style.

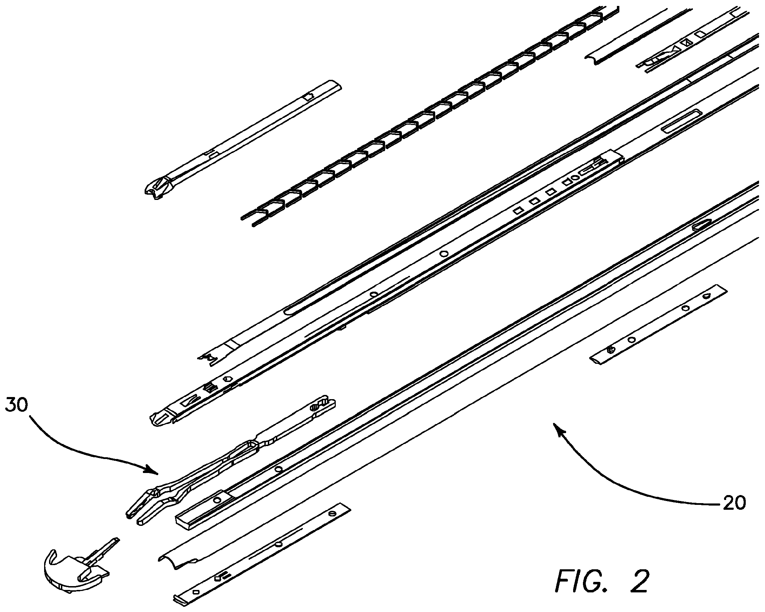

With reference to Figure 2 a distal end of the laparoscopic clip applier of Figure 1 is illustrated. As illustrated, the distal end of the laparoscopic clip applier comprises a laparoscopic shaft assembly 20 and jaw assembly 30. The shaft assembly 20 extends generally longitudinally from a proximal end at the handle assembly to a distal end. A jaw assembly 30 comprising a pair of opposing jaws is disposed at the distal end. A central portion of the shaft assembly extends between the proximal end and the distal end. An actuation assembly is positioned at the proximal end. The jaw assembly is configured to receive a surgical clip when the jaws are in an open configuration with one jaw spaced apart from an opposing jaw. Once the jaw assembly has received the surgical clip, the jaws are clamped to a closed configuration in which the jaws are approximated to clamp the surgical clip. The jaw assembly 30 can then be returned to the open configuration to receive another clip.

With continued reference to Figure 2, the central portion of the shaft assembly 20 comprises a generally tubular member extending from the proximal end to the distal end. The tubular member has a generally smooth outer surface to be positioned through a seal interface of a surgical access port such as a trocar cannula. A plurality of surgical clips can be positioned within the tubular member. Furthermore, the shaft assembly can comprise a mechanism for feeding the distal-most clip of the plurality of surgical clips to the jaw assembly and a mechanism for closing the jaws of the jaw assembly. The tubular member and jaw assembly can be sized for insertion through a surgical access port having a specific inner diameter. For example, the tubular member and jaw assembly can be sized for insertion through a 15mm, 12mm, 10mm, 8mm, or 5mm trocar cannula or a surgical access port having another inner diameter.

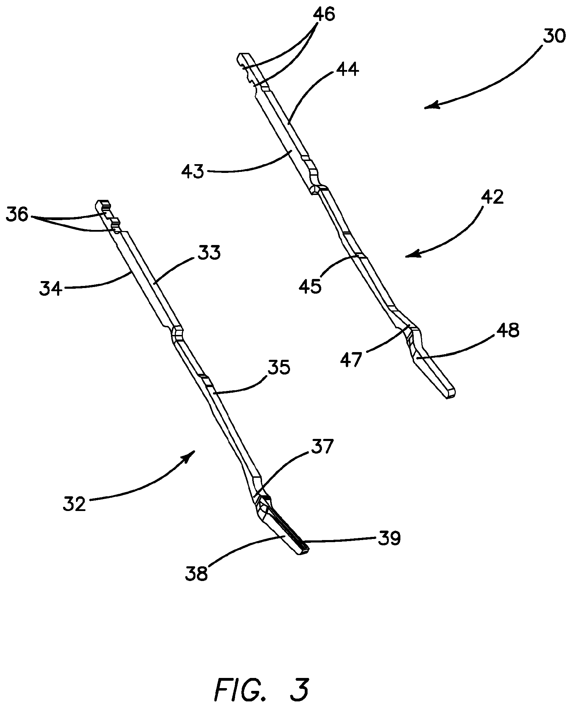

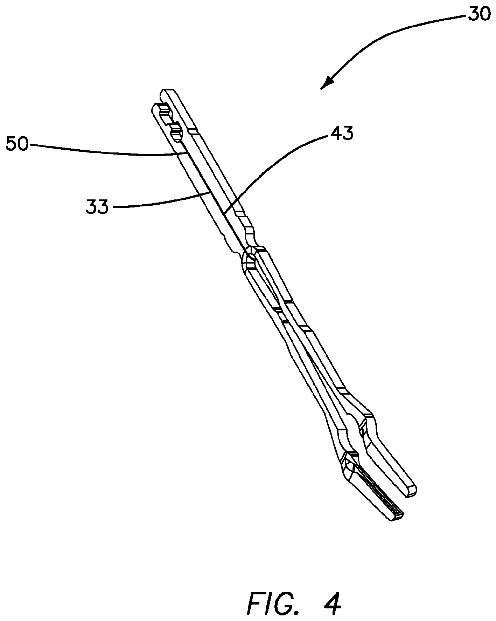

With reference to Figures 3-4, an embodiment of jaw assembly 30 for use with a laparoscopic clip applier such as that illustrated in Figures 1 and 2 is illustrated in a pre-assembly configuration (Figure 3) and an assembled configuration (Figure 4). As illustrated, the jaw assembly 30 comprises a first jaw 32 or jaw member and a second jaw 42 or jaw member that are each independently formed. The first jaw 32 and second jaw 42 are joined, such as by a welding operation creating a join or weld seam 52.

With continued reference to Figures 3-4, the first jaw extends generally longitudinally from a proximal end 34 to a distal end 38. A proximal base at the proximal end 34 can include one or more mating features such as notches or recesses 36 formed therein that can be sized and configured to couple the jaw assembly 30 to the shaft assembly of a laparoscopic clip applier. The proximal base at the proximal end can further comprise an interface surface 33 at a radially inwardly facing portion thereof that is configured to contact and be joined to the second jaw 42 when the fist jaw 32 and the second jaw 42 are assembled to form the jaw assembly 30.

With continued reference to Figures 3-4, the first jaw 32 can further comprise a first arm 35 extending distally from the proximal base at the proximal end to a clip jaw at the distal end. The first arm 35 can be configured to be flexible and provide a slight radially outward bias such that the clip jaw in an undisturbed state will be in a radially outward or open configuration but can be actuated to a closed or crimped configuration by a clip closure member. For example, in the illustrated embodiment, the first arm 35 has a radial outward bend at a proximal end thereof and the first arm 35 extends radially outwardly with respect to a longitudinal axis defined by the proximal base. Moreover, the first arm 35 has a lateral width that is small relative to a lateral width of the proximal base and a length along the longitudinal axis that is long relative to a length of the proximal base such that the distal end of the first arm 35 is flexible to allow movement of the clip jaw from an open configuration to a clip crimped configuration.

With continued reference to Figures 3-4, the first clip arm 35 can further comprise a first cam bend 37 at a distal end thereof. The first cam bend 37 can provide a cam surface for a longitudinally advanceable clip closure member to actuate the clip jaw from the open configuration to the clip crimped configuration. The clip jaw extends generally distally from the first cam bend 37. The clip jaw can desirably comprise a first clip channel or groove 39 formed therein and configured to receive a leg of a surgical clip therein.

With continued reference to Figures 3-4, in the illustrated embodiment, the second jaw 42 of the jaw assembly 30 is a mirror image of the first jaw. Accordingly, as illustrated, the second jaw 42 extends from a proximal end 44 to a distal end 48 and comprises one or more recesses 46 formed in a proximal base at the proximal end 44. The second jaw 42 can further comprise a second arm 45 having a radial outward bend at a proximal end thereof and a second cam bend 47 at a distal end thereof. A clip jaw having a clip groove therein is positioned at the distal end 48 of the second jaw. As illustrated in Figure 4, the interface surface 33 of the proximal base of the first jaw 32 can be positioned to contact the interface surface 43 of the proximal base of the second jaw 42 and the jaws 32, 42 joined. In certain embodiments, the jaws 32, 42 can be joined by a welding operation creating a weld seam 50.

While the illustrated embodiment of jaw assembly 30 includes a first jaw 32 and second jaw 42 that are mirror images of one another, it is contemplated that in other embodiments, one or both of the first jaw and second jaw can have geometric variations that can enhance joining thereof. For example, in some embodiments, the first jaw and second jaw can be configured to be press fit together. In other embodiments, the first jaw and second jaw can be configured with mating features such as a dovetail joint or 'puzzle piece' joint at a proximal end thereof.

While the first jaw 32 of the jaw assembly extends generally longitudinally from a proximal end 34 to a distal end 38, it is noted that certain features of the first jaw 32 deviate from this longitudinal configuration. For example, as previously discussed, the first arm 35 comprises a radially outward bend at a proximal end thereof, a cam bend at a distal end thereof, and a radially outward extent therebetween relative to the longitudinal axis. Moreover, the clip jaw can extend transverse to the longitudinal axis such that a clip will be positioned at a desired location relative to the laparoscopic shaft. Additionally, to facilitate reliable, repeatable clip loading and closure and avoid undesirable clip jams and drops, manufacturing processes for clip applier jaw assemblies have tended to require complex geometries having tight tolerances to be maintained throughout the manufacturing process. Thus, previous single-component jaw assemblies have required multiple manufacturing operations and post-processes. Advantageously, manufacturing a clip applier jaw assembly as two separate jaw components allows desirable jaw geometry to be achieved in a reliable, repeatable fashion with fewer manufacturing operations.

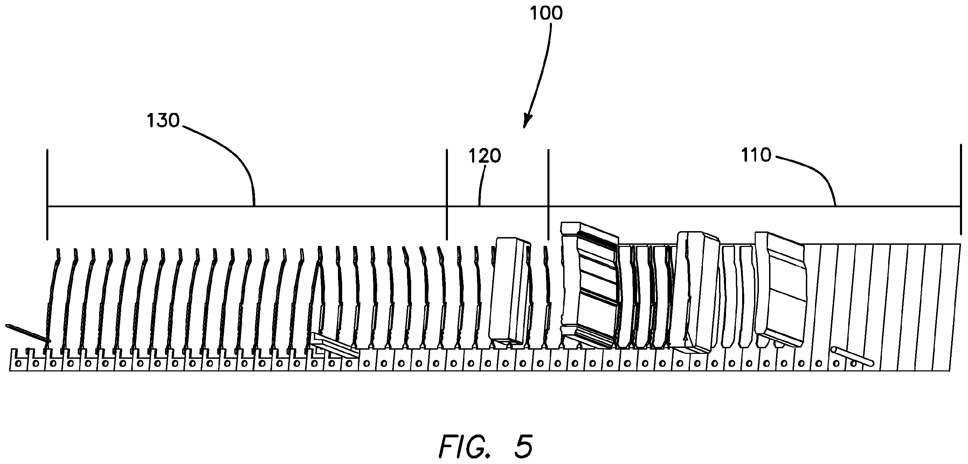

With reference to Figure 5, a schematic view of an exemplary manufacturing process for a jaw of a jaw assembly of a laparoscopic clip applier is illustrated. As illustrated, in one embodiment, a process 100 for manufacturing a jaw of a jaw assembly for a laparoscopic clip applier can comprise a progressive stamping process. The jaw manufacturing process 100 can comprise cutting operations 110, deburring operations 120, and finishing operations 130. Advantageously, these operations can be performed in a single die.

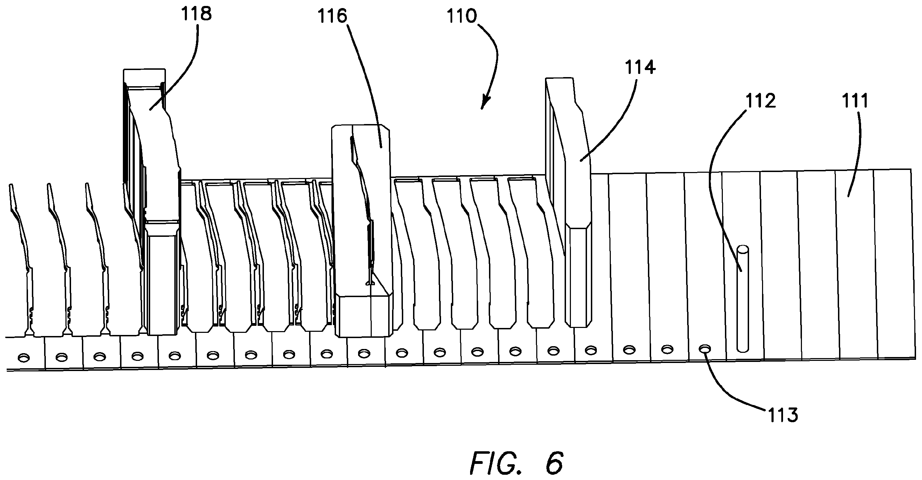

With reference to Figure 6, a schematic view of the cutting operations 110 of the manufacturing process 100 of Figure 5 is illustrated. As a sheet of material 111 enters the die, a first punching or cutting operation 112 is performed to create a pilot hole 113 that can be used to index or align the strip of material as it progresses through the various operations performed in the die. A second punching or cutting operation 114 is used to remove material from the sheet 111 to create a rough jaw blank which will be further cut and formed into a finished jaw. A third punching or cutting operation 116 is them performed on the rough jaw blank to create a detailed jaw blank including detailed jaw geometry. A fourth punching or cutting operation 118 is then performed to remove excess material from the detailed jaw blank. As illustrated, the progressive series of cutting operations allows a compound cut to be formed in the jaw blank having a detailed, multifaceted geometry having a plurality of edges and including relatively thin material sections. Advantageously, forming such a compound cut in a progressive stamping operation allows for a reduction in opposing stresses within the jaw as compared with a jaw assembly manufactured with previous processes.

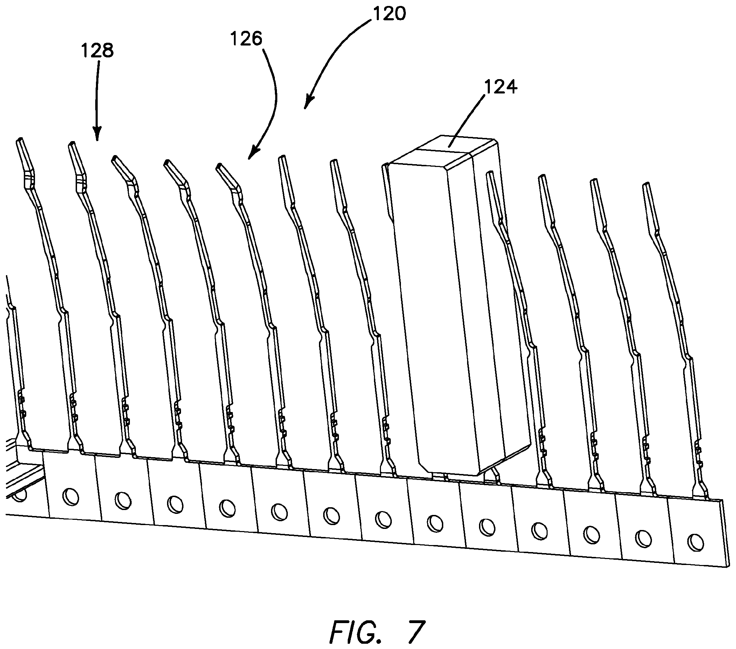

With reference to Figure 7, a schematic view of deburring operations 120 of the manufacturing process 100 of Figure 5 is illustrated. Once the cutting operations have created a detailed jaw blank, a perimeter/edge/profile coining process 124 can be performed to remove burrs or other excess material from the detailed jaw blank. Advantageously, this deburring process can be performed while the jaw blank is in the die, which can eliminate the need for a separate deburring process after stamping. In manufacture of previous single component jaw assemblies, a centrifugal tumbling process was typically used after stamping to deburr a jaw assembly. While performing deburring operations in the progressive stamping die as discussed herein is preferred, it is contemplated that in other implementations of the process described herein, such a separate deburring process could likewise be used. However, this separate centrifugal tumbling process could increase the time, cost, and manufacturing complexity of jaw assembly manufacture and could potentially result in manufacturing variations in the resulting jaws.

With continued reference to Figure 7, following the edge coining process 124, bending operations 126, 128 can be conducted to form the cam surface at the distal end of the arm and orient the clip jaw at a desired position relative to the arm of the jaw. In the illustrated embodiment a first bend 126 and a second bend 128 can form the cam surface and orient the clip jaw at a desired orientation. It is contemplated that in other implementations of the processes described herein, in order to manufacture clip jaws having different geometries, more or fewer than two bending operations can be performed to achieve a desired clip jaw geometry.

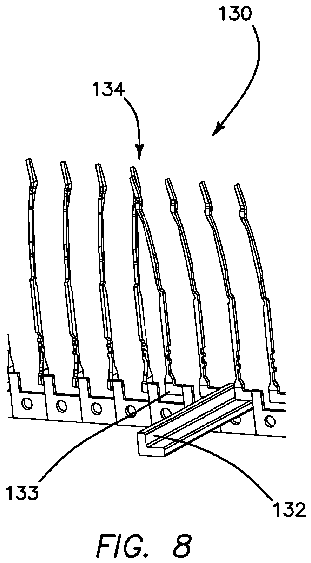

With reference to Figure 8, a schematic view of a first portion of the finishing operations 130 of the manufacturing process 100 of Figure 5 is illustrated. As illustrated, a carrier cutting or punching operation 132 is performed to remove material at a proximal end of the jaw blank, to form a carrier 133. An orienting or bending operation 134 is then performed within the die to reorient the carrier (and the attached jaw blank) relative to an unformed portion of the strip of material. As illustrated, the carrier and attached jaw blank is reoriented approximately 90 degrees relative to the unformed portion of the strip of material. Advantageously, by reorienting the carrier and jaw blank, a clip groove can be formed with a ram-driven coining operation with a direct vertical motion rather than a cam-driven operation with a vertical-to-horizontal transfer motion.

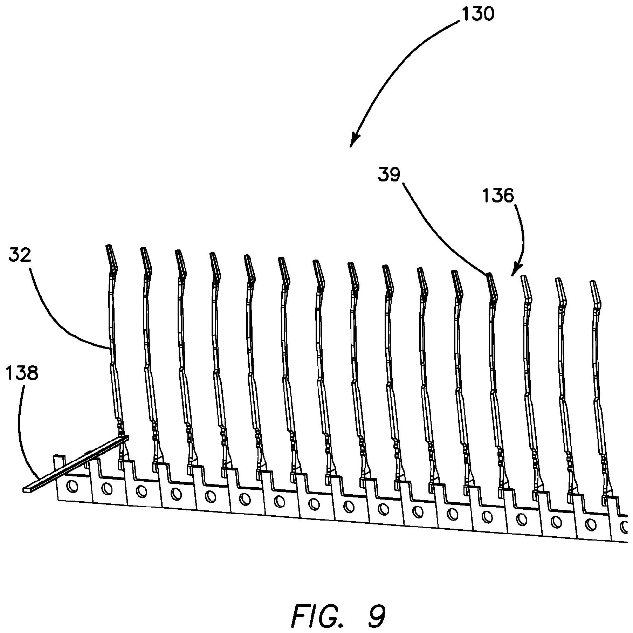

With reference to Figure 9, a schematic view of a second portion of the finishing operations 130 of the manufacturing process 100 of Figure 5 is illustrated. As illustrated, a coining operation 136 is performed to create the clip groove 39 in the clip jaw. As discussed above, the coining operation 136 can be carried out with a ram-driven process with a direct vertical motion. Once the clip groove has been formed, a cutting or punching operation 138 can be performed to separate the finished clip jaw 32 from the carrier.

With reference to Figures 5-9, advantageously all of the operations 110, 120, 130 described herein to form a single jaw of a jaw assembly can be repeatably, reliably performed in a single die. To form an embodiment of jaw assembly where each jaw is a mirror image of the other, each jaw will have a dedicated die. With previous single component jaw assemblies, typically initial cutting operations were performed in a first die, secondary operations, such as coining a clip groove, were performed in a secondary die, and post processing such as deburring by centrifugal tumbling was performed following the stamping processes. Advantageously, by forming substantially complete jaws in a single die per jaw, the manufacturing complexity, cost, and time can be significantly reduced as compared to previous methods.

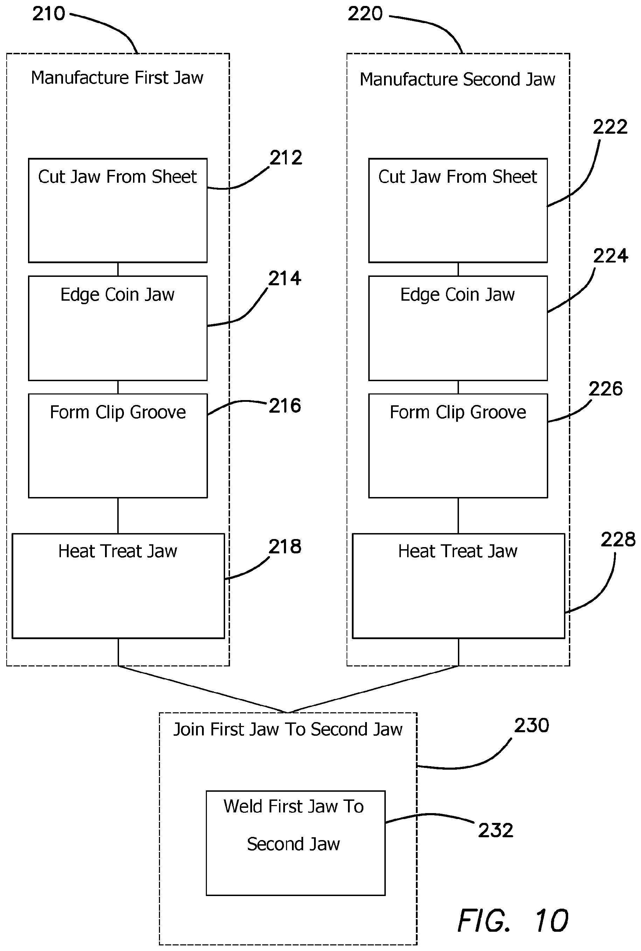

With respect to Figure 10, a schematic view of a flow chart illustrating an exemplary manufacturing process for a jaw assembly is illustrated. A first jaw can be manufactured by way of a process 210, such as that described herein and illustrated in Figures 5-9, including a cutting 212 a jaw from a sheet of material, edge coining 214 the jaw to deburr, and finishing the jaw, including forming 216 a clip groove. Once the first jaw has been formed, it can be processed such as by heat treating 218 to achieve desired material properties. Likewise, a second jaw can be manufactured by way of a similar process 220 that can include cutting 222, coining 224, and forming 226 operations and heat treating 228. The jaw assembly can then be formed by a joining 230 operation of the first jaw and a second jaw. In certain implementations of the manufacturing processes, a welding 232 operation such as laser welding is used to join the first jaw to the second jaw. Once the jaw assembly has been formed, further processing operations, such as passivation or surface treatments can be applied to achieve desired characteristics of the jaw assembly.

Although this specification discloses certain preferred embodiments and examples, it will be understood by those skilled in the art that alternative embodiments may be made which may fall within the scope of the present invention, as defined by the following claims.

## Claims

1. A jaw assembly (30) for a surgical clip applier, the jaw assembly (30) comprising:
a first jaw (32) having a proximal end (34) and a distal end (38), the first jaw (32) comprising a first base at the proximal end (34), the first base comprising a first interface surface (33) at a radially inwardly facing portion thereof;
a second jaw (42) having a proximal end (44) and a distal end (48), the second jaw (42) comprising a second base at the proximal end (44), the second base comprising a second interface surface (43) at a radially inwardly facing portion thereof; and
a welded seam (50) coupling the first jaw (32) to the second jaw (42);
wherein the first jaw (32) and the second jaw (42) are positioned such that the first interface surface (33) contacts the second interface surface (43) and the welded seam (50) couples the first jaw (32) to the second jaw (42) at the contact of the first interface surface (33) with the second interface surface (43).

2. The jaw assembly (30) of claim 1, wherein the first jaw (32) comprises a first jaw member extending distally from the first base to the distal end (38) of the first jaw, and
wherein the second jaw (42) comprises a second jaw member extending distally from the second base to the distal end (48) of the second jaw.

3. The jaw assembly of claim 2, wherein the welded seam (52) couples the first base to the second base.

4. The jaw assembly of claim 1, wherein the second jaw (42) comprises a mirror image of the first jaw (32).

5. The jaw assembly of claim 1, wherein the welded seam (52) comprises a laser welded seam.

6. A method of manufacturing the jaw assembly (30) of any of claims 1-5, the method comprising:
manufacturing the first jaw (32);
manufacturing the second jaw (42);
joining the first jaw (32) to the second jaw (42);
wherein the first jaw and the second jaw are positioned such that the first interface surface contacts the second interface surface and the first jaw is joined to the second jaw at the contact of the first interface surface with the second interface surface.

7. The method of claim 6, wherein manufacturing the first jaw (32) further comprises cutting the first jaw (32) from a sheet of material with a stamping process using a die.

8. The method of claim 7, wherein the first jaw (32) comprises a plurality of edges and the stamping process is configured to provide a compound cut defining the plurality of edges in a single operation on the die.

9. The method of claim 7, wherein manufacturing the first jaw (32) further comprises edge coining the first jaw (32) within the die after cutting the first jaw (32) from the sheet of material.

10. The method of claim 9, wherein manufacturing the first jaw (32) further comprises forming a clip groove (39) in the first jaw (32).

11. The method of claim 10, wherein forming the clip groove (39) in the first jaw (32) is performed in the die.

12. The method of claim 11, wherein forming the clip groove (39) in the first jaw (32) comprises moving an orientation of the first jaw (32) within the die and coining the clip groove (39).

13. The method of claim 10, wherein manufacturing the first jaw (32) further comprises heat treating the first jaw (32) once the clip groove (39) has been formed in the first jaw.

14. The method of claim 6, wherein the second jaw (42) comprises a mirror image of the first jaw (32).

15. The method of claim 6, wherein joining the first jaw (32) to the second jaw (42) comprises welding the first jaw to the second jaw.

16. The method of claim 15, wherein welding the first jaw (32) to the second jaw (42) comprises laser welding the first jaw to the second jaw.

17. A laparoscopic clip applier comprising:
an elongate shaft (20) having a proximal end and a distal end;
a handle assembly (10) disposed at the proximal end of the elongate shaft;
a jaw assembly (30) extending distally from the distal end of the elongate shaft, the jaw assembly actuatable by the handle assembly (10) to crimp a clip; wherein the jaw assembly (30) comprises:
the jaw assembly (30) of any of claims 1-5.

## Patentansprüche

1. Backenanordnung (30) für einen Applikator für chirurgische Klammern, wobei die Backenanordnung (30) umfasst:
eine erste Backe (32), aufweisend ein proximales Ende (34) und ein distales Ende (38), wobei die erste Backe (32) eine erste Basis an dem proximalen Ende (34) umfasst, wobei die erste Basis eine erste Grenzflächenoberfläche (33) an einem radial nach innen weisenden Abschnitt davon umfasst;
eine zweite Backe (42), aufweisend ein proximales Ende (44) und ein distales Ende (48), wobei die zweite Backe (42) eine zweite Basis an dem proximalen Ende (44) umfasst, wobei die zweite Basis eine zweite Grenzflächenoberfläche (43) an einem radial nach innen weisenden Abschnitt davon umfasst; und
eine Schweißnaht (50), die die erste Backe (32) an die zweite Backe (42) koppelt;
wobei die erste Backe (32) und die zweite Backe (42) derart positioniert werden, dass die erste Grenzflächenoberfläche (33) die zweite Grenzflächenoberfläche (43) berührt und die Schweißnaht (50) bei der Berührung der ersten Grenzflächenoberfläche (33) mit der zweiten Grenzflächenoberfläche (43) die erste Backe (32) an die zweite Backe (42) koppelt.

2. Backenanordnung (30) nach Anspruch 1, wobei die erste Backe (32) ein erstes Backenelement umfasst, das sich distal von der ersten Basis zu dem distalen Ende (38) der ersten Backe erstreckt, und
wobei die zweite Backe (42) ein zweites Backenelement umfasst, das sich distal von der zweiten Basis zu dem distalen Ende (48) der zweiten Backe erstreckt.

3. Backenanordnung nach Anspruch 2, wobei die Schweißnaht (52) die erste Basis an die zweite Basis koppelt.

4. Backenanordnung nach Anspruch 1, wobei die zweite Backe (42) ein Spiegelbild der ersten Backe (32) umfasst.

5. Backenanordnung nach Anspruch 1, wobei die Schweißnaht (52) eine Laserschweißnaht umfasst.

6. Verfahren zur Herstellung der Backenanordnung (30) nach einem der Ansprüche 1-5, wobei das Verfahren umfasst:
Herstellen der ersten Backe (32);
Herstellen der zweiten Backe (42);
Verbinden der ersten Backe (32) mit der zweiten Backe (42);
wobei die erste Backe und die zweite Backe derart positioniert werden, dass die erste Grenzflächenoberfläche die zweite Grenzflächenoberfläche berührt und die erste Backe bei der Berührung der ersten Grenzflächenoberfläche mit der zweiten Grenzflächenoberfläche mit der zweiten Backe verbunden wird.

7. Verfahren nach Anspruch 6, wobei das Herstellen der ersten Backe (32) ferner ein Schneiden der ersten Backe (32) aus einer Materialbahn mit einem Stanzvorgang unter Verwendung eines Stempels umfasst.

8. Verfahren nach Anspruch 7, wobei die erste Backe (32) eine Vielzahl von Kanten umfasst und der Stanzvorgang dazu konfiguriert ist, einen Schifterschnitt bereitzustellen, der die Vielzahl von Kanten in einem einzigen Arbeitsschritt an dem Stempel definiert.

9. Verfahren nach Anspruch 7, wobei das Herstellen der ersten Backe (32) ferner ein Kantenprägen der ersten Backe (32) innerhalb des Stempels nach dem Schneiden der ersten Backe (32) aus der Materialbahn umfasst.

10. Verfahren nach Anspruch 9, wobei das Herstellen der ersten Backe (32) ferner ein Formen einer Klammernut (39) in der ersten Backe (32) umfasst.

11. Verfahren nach Anspruch 10, wobei das Formen der Klammernut (39) in der ersten Backe (32) in dem Stempel durchgeführt wird.

12. Verfahren nach Anspruch 11, wobei das Formen der Klammernut (39) in der ersten Backe (32) ein Bewegen einer Ausrichtung der ersten Backe (32) innerhalb des Stempels und ein Prägen der Klammernut (39) umfasst.

13. Verfahren nach Anspruch 10, wobei das Herstellen der ersten Backe (32) ferner ein Wärmebehandeln der ersten Backe (32) umfasst, sobald die Klammernut (39) in der ersten Backe geformt wurde.

14. Verfahren nach Anspruch 6, wobei die zweite Backe (42) ein Spiegelbild der ersten Backe (32) umfasst.

15. Verfahren nach Anspruch 6, wobei das Verbinden der ersten Backe (32) mit der zweiten Backe (42) ein Schweißen der ersten Backe an die zweite Backe umfasst.

16. Verfahren nach Anspruch 15, wobei das Schweißen der ersten Backe (32) an die zweite Backe (42) ein Laserschweißen der ersten Backe an die zweite Backe umfasst.

17. Applikator für Klammern für eine Laparoskopie, umfassend:
einen länglichen Schaft (20), aufweisend ein proximales Ende und ein distales Ende;
eine Griffanordnung (10), die an dem proximalen Ende des länglichen Schafts angeordnet ist;
eine Backenanordnung (30), die sich distal von dem distalen Ende des länglichen Schafts erstreckt, wobei die Backenanordnung durch die Griffanordnung (10) betätigbar ist, um eine Klammer zu crimpen; wobei die Backenanordnung (30) umfasst:
die Backenanordnung (30) nach einem der Ansprüche 1-5.

## Revendications

1. Ensemble de mâchoire (30) pour un applicateur de clips chirurgicaux, l'ensemble de mâchoire (30) comprenant :
une première mâchoire (32) ayant une extrémité proximale (34) et une extrémité distale (38), la première mâchoire (32) comprenant une première base au niveau de l'extrémité proximale (34), la première base comprenant une première surface d'interface (33) au niveau d'une partie tournée vers l'intérieur de manière radiale de celle-ci ;
une deuxième mâchoire (42) ayant une extrémité proximale (44) et une extrémité distale (48), la deuxième mâchoire (42) comprenant une deuxième base au niveau de l'extrémité proximale (44), la deuxième base comprenant une deuxième surface d'interface (43) au niveau d'une partie tournée vers l'intérieur de manière radiale de celle-ci ; et
un joint étanche soudé (50) couplant la première mâchoire (32) à la deuxième mâchoire (42) ;
dans lequel la première mâchoire (32) et la deuxième mâchoire (42) sont positionnées de sorte que la première surface d'interface (33) entre en contact avec la deuxième surface d'interface (43) et le joint étanche soudé (50) couple la première mâchoire (32) à la deuxième mâchoire (42) au niveau du contact de la première surface d'interface (33) avec la deuxième surface d'interface (43).

2. Ensemble de mâchoire (30) selon la revendication 1, dans lequel la première mâchoire (32) comprend un premier élément de mâchoire s'étendant de manière distale de la première base à l'extrémité distale (38) de la première mâchoire, et
dans lequel la deuxième mâchoire (42) comprend un deuxième élément de mâchoire s'étendant de manière distale de la deuxième base à l'extrémité distale (48) de la deuxième mâchoire.

3. Ensemble de mâchoire selon la revendication 2, dans lequel le joint étanche soudé (52) couple la première base à la deuxième base.

4. Ensemble de mâchoire selon la revendication 1, dans lequel la deuxième mâchoire (42) comprend une image miroir de la première mâchoire (32).

5. Ensemble de mâchoire selon la revendication 1, dans lequel le joint étanche soudé (52) comprend un joint étanche soudé au laser.

6. Procédé de fabrication de l'ensemble de mâchoire (30) selon l'une quelconque des revendications 1 à 5, le procédé comprenant :
la fabrication de la première mâchoire (32) ;
la fabrication de la deuxième mâchoire (42) ;
la liaison de la première mâchoire (32) à la deuxième mâchoire (42) ;
dans lequel la première mâchoire et la deuxième mâchoire sont positionnées de sorte que la première surface d'interface entre en contact avec la deuxième surface d'interface et la première mâchoire est reliée à la deuxième mâchoire au niveau du contact de la première surface d'interface avec la deuxième surface d'interface.

7. Procédé selon la revendication 6, dans lequel la fabrication de la première mâchoire (32) comprend en outre la découpe de la première mâchoire (32) à partir d'une feuille de matériau avec un processus d'estampage à l'aide d'une matrice.

8. Procédé selon la revendication 7, dans lequel la première mâchoire (32) comprend une pluralité de bords et le processus d'estampage est configuré pour fournir une découpe composée définissant la pluralité de bords en une seule opération sur la matrice.

9. Procédé selon la revendication 7, dans lequel la fabrication de la première mâchoire (32) comprend en outre la frappe de bord de la première mâchoire (32) au sein de la matrice après la découpe de la première mâchoire (32) dans la feuille de matériau.

10. Procédé selon la revendication 9, dans lequel la fabrication de la première mâchoire (32) comprend en outre la formation d'une rainure de clip (39) dans la première mâchoire (32).

11. Procédé selon la revendication 10, dans lequel la formation de la rainure de clip (39) dans la première mâchoire (32) est réalisée dans la matrice.

12. Procédé selon la revendication 11, dans lequel la formation de la rainure de clip (39) dans la première mâchoire (32) comprend le déplacement d'une orientation de la première mâchoire (32) au sein de la matrice et la frappe de la rainure de clip (39).

13. Procédé selon la revendication 10, dans lequel la fabrication de la première mâchoire (32) comprend en outre le traitement thermique de la première mâchoire (32) une fois que la rainure de clip (39) a été formée dans la première mâchoire.

14. Procédé selon la revendication 6, dans lequel la deuxième mâchoire (42) comprend une image miroir de la première mâchoire (32).

15. Procédé selon la revendication 6, dans lequel la liaison de la première mâchoire (32) à la deuxième mâchoire (42) comprend le soudage de la première mâchoire à la deuxième mâchoire.

16. Procédé selon la revendication 15, dans lequel le soudage de la première mâchoire (32) à la deuxième mâchoire (42) comprend le soudage au laser de la première mâchoire à la deuxième mâchoire.

17. Applicateur de clips laparoscopiques comprenant :
un arbre allongé (1908) ayant une extrémité proximale et une extrémité distale ;
un ensemble de poignée (10) disposé au niveau de l'extrémité proximale de l'arbre allongé ;
un ensemble de mâchoire (30) s'étendant de manière distale à partir de l'extrémité distale de la tige allongée, l'ensemble de mâchoire pouvant être actionné par l'ensemble de poignée (10) pour sertir un clip ; dans lequel l'ensemble de mâchoire (30) comprend :
l'ensemble de mâchoire (30) selon l'une quelconque des revendications 1 à **5.**
